Europäisches Patentamt

European Patent Office

Office européen des brevets

(19)

(11) EP 0 509 989 B1

(12) EUROPEAN PATENT SPECIFICATION

(45) Date of publication and mention
of the grant of the patent:
**27.03.1996 Bulletin 1996/13**

(21) Application number: **90907615.0**

(22) Date of filing: **08.01.1990**

(51) Int Cl.$^6$: **C12N 9/22**

(86) International application number:
**PCT/US90/00141**

(87) International publication number:
**WO 91/10730 (25.07.1991 Gazette 1991/17)**

(54) **METHOD OF PREPARING RIBONUCLEASE DIMERS**

VERFAHREN ZUR HERSTELLUNG VON RIBONUKLEASE-DIMEREN

PROCEDE DE PREPARATION DE DIMERES DE RIBONUCLEASE

(84) Designated Contracting States:
**AT BE CH DE DK ES FR GB IT LI LU NL SE**

(43) Date of publication of application:
**28.10.1992 Bulletin 1992/44**

(73) Proprietor: **NIKA HEALTH PRODUCTS LIMITED**
**FL-9490 Vaduz (LI)**

(72) Inventors:
• **HERRMANN, Peter**
**CH-4452 Itingen (CH)**

• **KLEIN, Peter**
**CH-4452 Itingen (CH)**

(74) Representative: **Büchel, Kurt F., Dr.**
**Patentbüro Büchel & Partner AG**
**Letzanaweg 25-27**
**FL-9495 Triesen (LI)**

(56) References cited:
**WO-A-89/11294**

• **EUROPEAN JOURNAL OF BIOCHEMISTRY,**
**vol. 124, no. 1, 1982, Berlin (DE); S.**
**SORRENTINO et al., pp. 183-190**
• **BIOCHEMICAL JOURNAL, vol. 173, 1978,**
**London (GB); J. CARLSSON et al., pp. 723-737**

## Description

Field of the Invention

The invention relates to a series of techniques used in the preparation and purification of a ribonuclease dimer which can be used in the treatment of viral and bacterial infections.

Background of the Invention

The ever-growing number of bacterial strains and viral diseases which are resistant to known antibiotics have made it necessary to introduce new types of drugs in order to treat humans and animals. Among the many present treatments and medicines currently used, it has been known to administer enzymes in monomeric form in order to benefit patients afflicted with various diseases. In some cases, this is necessary because certain disease conditions cause a reduction in activity in certain enzymes. Enzymes are catalytically active proteins which perform almost all major life processes in organisms. Thus, many enzymes, either individually or in certain combinations, have been isolated for their physiochemical, physiological, or biological effects.

Among the various enzymes which are known to have therapeutic effects or which undergo a reduction of activity under certain disease conditions are the ribonucleases. The ribonucleases are a group of nucleic enzymes commonly found in many animal and plant organisms. The study of the properties of ribonuclease and methods of isolating this enzyme were initiated in the mid-1950's by Schmidt and McDonald. Among the many findings based on this enzyme, it was found that cancerous tissue had a greatly reduced activity of its ribonucleases. In one case, it was discovered that leukemic mice had a considerable decrease in acid ribonuclease activity, which was observed particularly in mitochondria and microsome fractions obtained from the spleen tissue of those animals. Studies such as these have indicated that the administration of ribonuclease could possibly be used to prevent or combat the symptoms associated with viral leukemia.

Unfortunately, the potential beneficial effects that could be achieved through use of ribonuclease have not materialized primarily due to the severe cytotoxic effect of the monomeric form of this enzyme. For example, in tests with cultured fibroblasts, there has been an observed cytotoxic effect from doses of ribonuclease in monomeric form even when administered at extremely small levels. Clearly, it was necessary to develop ways of maximizing the potential beneficial effects from ribonuclease while minimizing the potentially harmful cytotoxic effects associated with the monomeric form of the enzyme.

It has recently been discovered that an antiviral or antibacterial composition can be constructed from ribonuclease that does not exhibit cytotoxic effects when one prepares a composition based on the dimeric form of the enzyme. Use of ribonuclease dimer compositions has been found to be effective in the treatment of a number of infectious diseases, yet at the same time does not cause the highly cytotoxic effects normally associated with the ribonuclease monomer. The use of ribonuclease dimers in various therapeutic treatments is disclosed in a co-pending application, PCT Application US88/01785.

Although some ways of manufacturing the dimeric form of ribonuclease from its monomeric form are known, it is now important that one be able to produce large amounts of the dimeric form in an inexpensive and efficient manner. It is thus highly desirable to develop a system for producing and testing large amounts of purified ribonuclease dimer which has minimal amounts of impurities such as monomers, multimers, or other contaminants.

Summary of the Invention

In accordance with the present invention, an efficient method of preparing a purified ribonuclease dimer product is provided which comprises the steps of:

a) preparing a ribonuclease solution by adding monomeric ribonuclease to a buffer solution and adjusting the pH to at least about 9;
b) adding a suberimidate coupling reagent such as dimethyl suberimidate in order to dimerize the ribonuclease monomers in the ribonuclease solution while the solution is kept at a pH at or above about 9;
c) lowering the pH to about 7 in order to stop the dimerization reaction at a given point;
d) purifying the dimerized ribonuclease solution by carrying out a first filtration step in which the solution is eluted through an ion exchange resin, and the fractions containing a substantial amount of the dimeric form of ribonuclease are collected;
e) carrying out a second filtration step in which the collected fractions from the first filtration step are eluted through an ion exchange resin; and
f) collecting the highly purified dimeric ribonuclease product resulting from the second filtration step.

Using the above method, large amounts of purified ribonuclease dimer product can be manufactured, and this product will be extremely useful in treating a variety of viral and bacterial diseases.

Brief Description of the Drawings

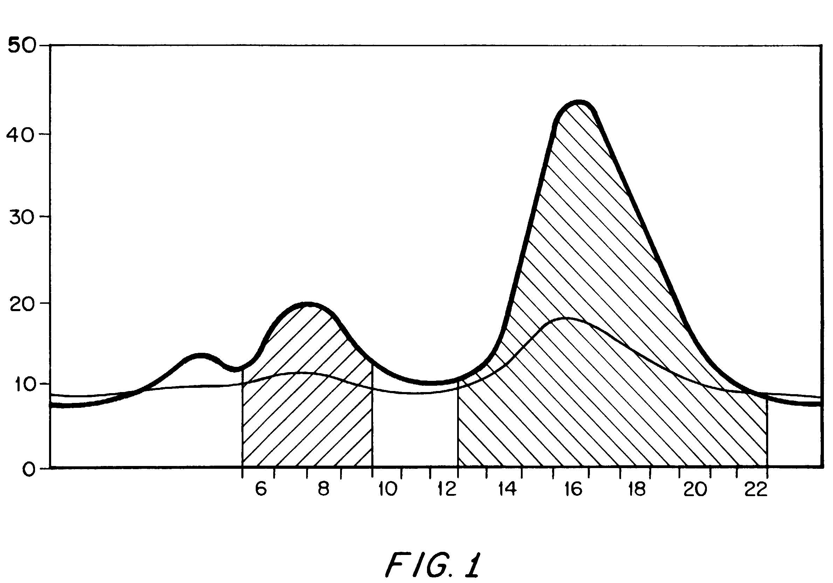

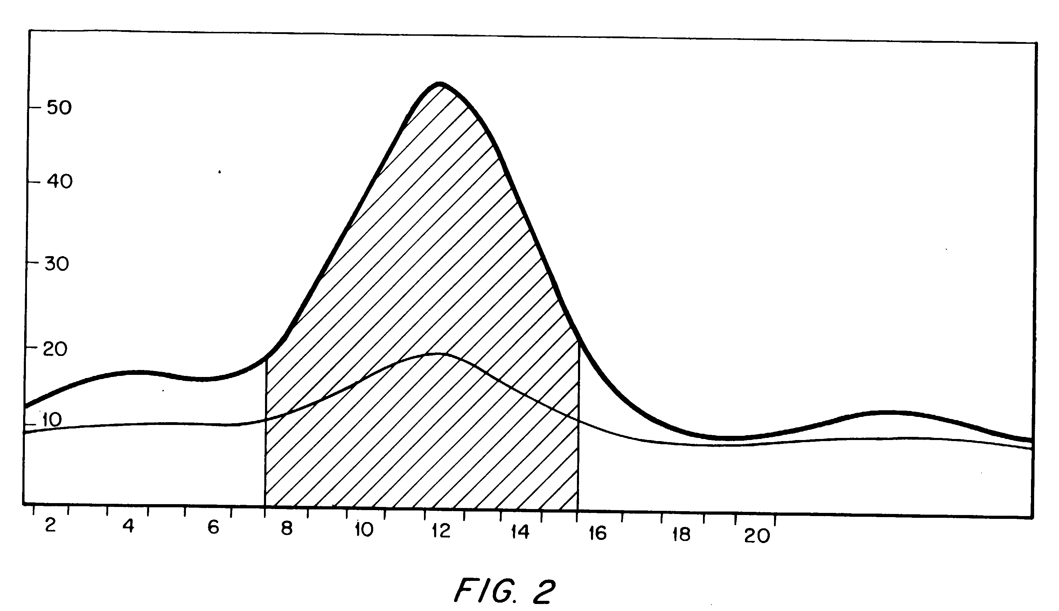

Figures 1 and 2 are graphic representations of elution profiles obtained via ion exchange chromatography carried out in accordance with the present invention.

Detailed Description of the Preferred Embodiments

In preparing the dimeric form of ribonuclease in accordance with the present invention, any currently available ribonuclease monomers can be used as the starting materials. In the present invention, the ribonuclease monomers used were obtained from Serva Feine Biochemica, GmbH, Heidelberg, and had catalog No. 28260. The monomers used can be comprised of Ribonuclease A, or any other of the various ribonucleases available. The ribonuclease monomers are prepared for the coupling reaction by dissolving the ribonuclease monomers in a phosphate buffer at room temperature in order to prepare a ribonuclease monomer solution. The buffer employed is preferably an 0.1 M disodium hydrogen phosphate dihydrate buffer, and the monomer is dissolved by continuously stirring the solution for at least about 2 hours. A suitable buffer solution has been prepared by dissolving about 70 grams of disodium hydrogen phosphate dihydrate in about 1,000 ml $H_2O$. Although actual amounts of the reagents and solutions used in the present invention can vary, the dimerization reaction of the present invention has been carried out using from 40 to about 60 grams of the ribonuclease monomer dissolved in a beaker containing about 5 liters of the disodium hydrogen phosphate dihydrate buffer. It is also preferred that the monomeric solution be adjusted to have a pH of at least about 9, and most preferably about 10. The pH adjustment can be made using 0.1 N NaOH.

The dimerization reaction is allowed to take place by adding a suberimidate coupling reagent in proportion to the ribonuclease monomer solution while maintaining the pH at or above at least about 9, and preferably 10 (+ or - 0.2). It is preferred that a dimethyl suberimidate coupling reagent be used in the present invention, but other known suberimidate coupling reagents can be employed. To the solution prepared as indicated above, 4-6 grams of dimethyl suberimidate is preferably added to the monomeric solution, and the coupling reagent will dissolve within about one minute. With continuous stirring by means of a magnetic stirrer or other device, the dimerization reaction is allowed to take place for about 30-60 minutes at room temperature of about 25°C (+ or - 5°C). The reaction can be stopped by lowering the pH to 7.0 (+ or - 0.2), and this can be accomplished by the addition of a 1 M HCl solution. At this point, it is preferred that the resulting mixture be concentrated using a tangential flow system, as will be described in more detail below.

At various points in time following the start of the coupling reaction, it is helpful to remove test samples from the solution and subject them to gel-electrophoresis analysis. Gel-electrophoresis studies have shown that although the monomeric form of the enzyme was clearly visible in almost every fraction analyzed, a band corresponding to the dimeric form of ribonuclease is recognizable after about 10 minutes of reaction time. A band corresponding to the dimeric form of the enzyme can be seen clearly after about 15 minutes, and this band becomes more pronounced as the reaction progresses. Oligomeric forms of the enzyme can also be observed after about 30 minutes following the addition of the coupling reagent. The monomeric form of ribonuclease has a relative molecular weight of 15,000, and the dimeric form will be double this size.

In order to prepare large amounts of a product which is primarily a purified ribonuclease dimer, it is preferred that the dimerized ribonuclease solution undergo at least two filtration steps in order to isolate the ribonuclease dimers and remove the ribonuclease in monomeric or multimeric form. The removed undimerized ribonuclease monomers can thus be collected and are preferably recycled into the present invention so as to further maximize the efficiency in the production of the dimeric form of the enzyme.

In order to separate out the oligomers from the dimerized ribonuclease solution, it is preferred that the solution be filtered through an ion exchange resin. In the preferred embodiment, the filtration is carried out using a column prepared with a Sephadex ion exchange material. A Sephadex column of about 25 cm in diameter and about 120 cm in height was prepared and rinsed with a volume of about 60 liters of 50 mM ammonium hydrogen carbonate at a pH of about 8.6. This carbonate buffer was prepared by dissolving about 4 grams of ammonium hydrogen carbonate in 1,000 ml of water. The entire ribonuclease solution obtained above is then applied to the column and eluted with an equilibrium buffer. Fractions of the solution are collected by a fraction collector, such as an LKB 2211 Suprec, and the flow rate of the solution is roughly 80 ml per minute. The elution profile is recorded by a recording device such as an LKB 2210 recorder measuring at a velocity of 0.5 mm/minute, and the absorption can be measured at 280 nm by an LKB 2238 UVICOR SII.

Elution profiles obtained from this filtration step will generally show three peaks: the first peak represents the multimers of ribonuclease, the second peak represents the dimers, and the last peak corresponds to the remaining amounts of the monomeric form of the enzyme. At this point it is also advisable to analyze the individual collected fractions using a gel-electrophoresis analysis. Such an analysis can be carried out by the SDS-polyacrylamide gel-electrophoresis process (or SDS-PAGE) as described in Thomas et al, PNAS 72:2626 (1975). In this electrophoretic study, preferably approximately 50 µl of each fraction collected is mixed with about 50 µl of a coating buffer and heated for a period of about 10 minutes at about 95°C. Next, about 25 µl of this mixture is spread into a gel pocket. Finally, the electrophoresis is carried out for approximately 4 hours at 20-35 mA in order to separate out the various forms of the ribonuclease enzymes. The protein bands are made visible by coloring with a dye such as Coomassie-Blue R250 (Merck).

Using this SDS-PAGE electrophoresis process, fractions containing the monomeric, dimeric, or oligomeric forms of ribonuclease, or mixtures of these, can be identified and sorted out. After identifying the fraction compositions, those fractions which are now mostly in the dimeric form are isolated and collected. The collected dimer fractions are preferably further concentrated down to about 800 ml in a tangential flow system. The membrane diaphragm of the tangential flow system can be rinsed again with a suitable solvent so that a greater amount of the dimer can be retained. This solution is preferably lyophilized and can be stored at temperatures of about 4°C until further reprocessing steps take place. Ideally, the lyophilization is carried out by distributing approximately 200 ml portions of the solution in 500 ml round bottom flasks and placed in a liquid nitrogen atmosphere in a rotation evaporator. The flasks are sealed shut and stored for 30 minutes at -30°C, after which the material is lyophilized.

Since the starting materials can often be expensive, it is particularly preferred that the monomers filtered out in the previous filtration step can be drawn back again into the dimerization process of the present invention. This can be carried out by collecting the fractions identified as containing a substantial amount of monomeric ribonuclease, concentrating them using a tangential flow system, and adding the coupling reagent identified above. The coupling reaction occurs as indicated above, and the filtration step described above is preferably carried out as well. After lyophilization of the recycled ribonuclease, the newly formed dimers are mixed thoroughly with the dimerized product obtained from the first dimerization cycle. Increases in the yield of the dimer product of up to 30% can be obtained by means of these recycling techniques.

The primarily dimeric solutions obtained above are further purified by carrying out a second filtration step in order to further separate out the dimers from monomers and oligomers and increase the purity of the final product. In this case, it is preferred that the lyophilized enzyme mixture obtained above is eluted through an ion exchange resin such as a Sephadex G 50 F column. It is also preferred in this step that the ion exchange column be adjusted to have a pH of about 5, and this can be achieved by the use of an 0.2 M sodium acetate buffer. The sodium acetate buffer of pH 5 has been prepared using about 27 g of sodium acetate trihydrate dissolved in 1,000 ml of water.

The collected fractions corresponding to the second peak, as determined through the measurements of the first filtration step, are applied to the Sephadex column. The conditions and materials described in the first filtration step are preferably employed again when the fractions undergo the second filtration step. Again, an elution profile is recorded and used in the identification and isolation of the desired fractions collected following the second filtration step. In this case, the elution profile shows a very large diner-containing main peak and only two weak secondary peaks.

It is again preferred that an SDS-PAGE analysis of the ribonuclease enzymes taken from the collected fractions be carried out as indicated above. These studies indicate that the fractions corresponding to the main peak of the elution profile contain highly purified ribonuclease dimer product. At this point, the fractions of the main peak are collected and preferably are concentrated by means of a tangential flow system. It is also preferred at this point that a desalination process be carried out by passing the fractions over a Sephadex G25 column. A column of about 25 cm in diameter and 65 cm in height with a base volume of 32 liters was operated at a flow rate of 15 liters per hour in order to carry out the desalination process. Additionally, a 50 mM ammonium hydrogen carbonate buffer was used.

The product at this point will consist of a highly purified ribonuclease dimer composition. However, in order to make lyophilized ribonuclease dimer into a pharmaceutically useful form, it is recommended that the dimers undergo a procedure wherein endotoxins can be removed. It is thus preferred that the purified dimer product obtained above be further eluted through a chromatographic means such as Detoxigel which has a high binding capacity for endotoxins. Detoxigel has an approximate binding capacity for endotoxins of about 2 mg/ml. Since non-specific binding with other components such as the protein dimers, is possible, maximum reclamation of the dimeric material can be obtained by the use of buffers in the physiological pH range. The addition of 0.1-0.5 M salt solution (e.g., NaCl) provides the necessary pH range in order to obtain a maximum dimer yield. Typically, a solution containing 1,500 E.U./ml when chromatographed on a detoxification column can be reduced to less than 1.0 E.U./ml.

After freeing the ribonuclease dimer solution of endotoxins, the solution is collected, sterile-filtered, and passed for lyophilization in a sterile round-bottom flask. The endotoxin content of the final preparation can be determined with a diagnostic kit such as "Coatest (R)" of the Kabi-Vitrum firm of Munich. This test is based on the activation of Limulus-Amoebocyten-Lysat (LAL) by the endotoxins. The activated LAL separates the yellow dye p-nitroanilin from a chromogenic substrate (S-2423), of which the extinction is measured as the determinant of the endotoxin content at 405 nm using a spectral photometer. If the endotoxin content is below 0.1 ng/mg, then the dimeric solution is placed in the round-bottom flasks and lyophilized.

The above process can be repeated as often as possible until the desired amount of final ribonuclease dimer product is obtained. Individual dimer batches after purification can be sterilized or further lyophilized as needed, and homogenous charges of ribonuclease dimer will be obtained which can be highly useful in the treatment of many viral and bacterial diseases, such as disclosed in co-pending application PCT/US88/01785. In addition to antiviral and antibacterial effects, other therapeutic benefits from ribonuclease dimers, such as use as an anti-inflammatory or anti-histaminic agent, can also be obtained without the potential cytotoxicity associated with the monomeric form of the enzyme. The present method is particularly useful in that large amounts of the purified beneficial ribonuclease dimer product can be efficiently produced. The particular ribonuclease dimer products obtained using the method of the present invention can be tested for its enzyme activity on ribonucleic acid solutions and monitored using a spectral photometer after the ribonuclease dimer is applied. Use of the present method thus allows for the preparation of a highly purified dimeric enzyme and a test of its activity as well.

The following examples are presented as illustrative of the present invention and are not intended to limit its scope in any way:

Example 1: Preparation of Ribonuclease Dimer

50 grams of ribonuclease monomer were dissolved in a covered beaker containing 5 liters of 0.1 M disodium hydrogen phosphate dihydrate and adjusted to pH 10.0 with 0.1 N NaOH. The disodium hydrogen phosphate dihydrate buffer was prepared by dissolving 70.98 g disodium hydrogen phosphate dihydrate in 1,000 ml of water. Next, 5 grams of the coupling reagent dimethyl suberimidate (Sigma Batch No. 29,3687) were added at 25°C (room temperature), and the dimethyl suberimidate dissolved within 1 minute. The pH level of the solution was controlled and adjusted continuously to pH 10 using 0.1 N NaOH. Using a magnetic stirrer, the solution was continuously stirred and allowed to react for 45 minutes at about 25°C. The reaction was stopped as the pH was lowered to about 7.0 using 1 M HCl.

At various points in time following the start of the coupling reaction, test samples were removed and were analyzed through gel-electrophoresis. Monomers, dimers, and multimers of the ribonuclease were separated out in the SDS gel. The monomeric form of ribonuclease used had a relative molecular weight of 15,000, and the dimeric form was of about double this size. The electrophoretic studies revealed that a dimeric band was seen to form clearly after 15 minutes following the addition of the coupling reagent, and this band became bolder as the reaction progressed. Additionally, oligomeric forms of ribonuclease were generated after 30 minutes.

The dimerized ribonuclease solution was concentrated to 800 ml in a tangential flow system (Millipore). The Millipore system included a Filtron Ausschluss 10,000d filter, an olefin membrane of 7 bar tear-resistance, and a Verder 80W Type 20-30 #60079 pump. The input pressure was 2 bar and the output pressure was at minimum lower than 0.2. The working capacity was approximately 1 liter per hour. The apparatus has a dead volume of 400 ml so that upon termination of the concentrating process, the apparatus is rinsed with 400 ml which leads to a final volume of 1,200 ml.

The concentrated ribonuclease solution is then subject to a filtration process using Sephadex G 50 F (Pharmacia) in order to separate out the various oligomers formed during the dimerization process. For this filtration, a column of 25.2 cm diameter and 120 cm height was rinsed with a volume of 60 liters of 50 mM ammonium hydrogen carbonate at pH 8.6. The 50 mM ammonium hydrogen carbonate buffer was prepared by dissolving 3.95 g ammonium hydrogen carbonate in 1,000 ml of water. Next, the entire protein solution of 1,200 ml was coated onto the gel bed and eluted with the equilibrium buffer. Fractions of approximately 1,000 ml were collected by a fraction collector (LKB 2211 Suprec) for approximately 15 minutes, corresponding to a flow rate of 80 ml/minute. The elution profile was recorded by an LKB 2210 recorder at a velocity of 0.5 mm/minute and the absorption was measured at 280 nm by LKB 2238 UVICOR SII. The elution profile as observed in Figure 1 shows three peaks, the first of which represents the multimers of ribonuclease, the second indicates the dimers, and the last peak corresponds to the monomeric forms of the enzyme. In Figure 1, the thick line indicates the absorption profile of the ribonuclease solutions with more sensitive detection than in the lower thin line of the graph.

The ribonuclease mixture in the individual fractions was then analyzed by the SDS-polyacrylamide-gel-electrophoresis (SDS-PAGE) procedure in accordance with Thomas et al, PNAS 72:2626 (1975). In this procedure, 50 µl of each fraction is mixed with 50 µl of a coating buffer and the mixture is heated for 10 minutes at 95°C. Next, 25 µl of this mixture is spread in a gel pocket. During the testing, the standard IV (Merck) protein mixture is spread on as a reference, covering molecular weights of 12, 300; 30,000; 45,000; 66,200; and 76,000. The coating buffer used was comprised of the following ingredients:

0.72 g tris HCL (0.06 M)
0.136 g EDTA (III) (5 mM)
0.18 g Glycerine (10%)
5 g SDS, pH set at 7.2 (add 90 ml water)
10 ml beta-mercaptoethanol (10%)

For the gel-electrophoresis procedure, an 18% separating gel is prepared which is overlaid with a 3.9% collecting gel. The separating gel solution for 18% acrylamide was comprised of the following ingredients:

9 g acrylamide
0.045 g bisacrylamide
0.136 g tris HCL pH 8.8 (0.325 M)
0.03 g SDS
200 µl 10% ammonium persulfate solution
20 µl TEMED

The collecting gel solution for 3.9% acrylamide was comprised of the following:

0.39 g acrylamide
10.4 mg bisacrylamide
0.125 m tris HCL pH 6.8
10 mg SDS
100 µl 10% ammonium persulfate solution

10 µl TEMED

The SDS-polyacrylamide gel was prepared by taking two 20 x 20 cm glass plates which are thoroughly cleaned and rinsed with ethanol, and placing them one atop the other. Two spacing strips of 1 mm thickness (length 20 cm, breadth 1 cm) provided the space between the plates into which the gel was poured. The spacing strips are mounted on the left and right edges of the glass plates. The bottom edge is sealed by a textile adhesive strip, and all three edges are reinforced with clamps. The edges are additionally sealed with a 1% agarose solution. After hardening of the agarose, the separating gel solution prepared above is filled into the interstices in vertical position up to approximately 3 cm below the top edge of the glass plate, and with the aid of a Pasteur pipette is overlaid with a layer of water,. The gel is polymerized after approximately 30 minutes. The coating of water is poured off, and the edge of the gel is rinsed one time with the collecting gel solution described above.

Next, the collecting gel solution is filled in up to the edge, and a Teflon sample collecting comb is put in so that the bottom edge of the sample pocket lies approximately 1 cm over the front edge of the separating gel layer. After approximately 15 minutes, the collecting gel is polymerized, and the comb can be removed. Next, the textile adhesive strip is removed and the gel is joined in the vertical position to an electrophoresis apparatus. The buffer chambers are filled with an electrophoresis buffer (6g tris-base, (0.05 M); 28.5 g glycine, (0.38 M); 1 g SDS, (0.1%); and 1,000 ml $H_2O$) and the gel pockets are rinsed once with the aid of a buffer spray. The ribonuclease dimer samples are heated for approximately 10 minutes in the buffer coating at 95°C and filled into a container or area in the testing sample pocket corresponding the gel pocket.

The electrophoresis was carried out at about 20 mA for about 4 hours. If overnight electrophoresis is desired however, this could be carried out at a reduced charge of about 6-8 mA. The moving front is made visible by intermixing of 0.02% bromophenol-blue into the coating buffer. The electrophoresis was terminated when the moving front of the process reached the bottom edge of the gel. The separating gel is cut out, dyed for approximately 30 minutes in a fixing solution and is then bleached again for 2 hours in a bleaching solution of 400 ml methanol, 140 ml acetic acid, and 2,000 ml $H_2O$. The fixing solution was prepared by combining 500 ml of the bleach solution with 12 ml of a dye solution comprising 1 g Coomassie-blue (R250, Merck), 50 ml $H_2O$, and 50 ml methanol. The protein bands were then made visible by coloring with the dye solution as indicated above. The traces made using the SDS-PAGE technique indicated a gradual increase in the amount of dimerized ribonuclease in the samples which ultimately included fractions containing multimers as well.

All of the fractions, which were mainly in dimeric form, were collected and concentrated to 800 ml in a tangential flow system (Millipore). The membrane diaphragm was rinsed again with 400 ml of the permeate so that a total volume of 1,200 ml of solution with the dimers remains leftover. Next, this solution was lyophilized and stored at 4°C until the next step. The lyophilization was carried out by distributing the solution in approximately 200 ml portions into 500 ml round-bottom flasks. The flasks were then placed in liquid nitrogen and frozen into a rotation evaporator (Heidolph VV60). The flasks were then sealed shut and stored for 30 minutes at -30°C. Finally, this material was lyophilized using standard operating procedures.

Before the next step of the process, monomeric ribonuclease filtered out from the first filtration step were collected and recycled into the dimerization process. In this case, the fractions from the filtration step which were primarily ribonuclease in monomeric form were collected and concentrated to 4 liters using the above-identified tangential flow system. Next, the monomeric fractions were dimerized using the coupling reagent as indicated above. Following the coupling step using the recycled monomers, a filtration process was carried out as indicated above, and the recycled monomers were now obtained in dimeric form. The lyophilisates obtained from the first dimerization process are combined with the dimeric lyophilisates obtained from recycling the monomers.

The combined lyophilized ribonuclease mixture obtained from the above steps is now chromatographed again over Sephadex G50F in order to obtain improved separation of the dimers from the monomers and the oligomers. The chromatographic procedure was carried out using a Sephadex G 50 F (Pharmacia) column in 0.2 M sodium acetate buffer at pH 5. The 0.2 M sodium acetate buffer was prepared by dissolving 27.22 g sodium acetate trihydrate in 1,000 ml of water. The chromatography was carried out under conditions and using the apparatus as identified above in the first filtration step.

In this case, an elution profile was again obtained, and this can be observed in Figure 2. In this figure, the thick line represents the absorption profile of the ribonuclease solutions obtained using more sensitive detection than in the profile indicated by the thin line. The middle of the more sensitive elution profile shows a large dimer-containing main peak, and only two weak secondary peaks. An SDS-PAGE analysis of the enzymes taken from the peak fractions shows a greater accumulation of the dimers in the main peak as compared with the elution profile of the first filtration step.

The fractions of the main peak were collected and concentrated by means of the tangential flow system described above. For desalination, these collected fractions were passed over a Sephadex G25 column. The column had a diameter of 25.2 cm, a height of 65 cm, a base volume of 32 liters, and was operated at a flow rate of 15 liters/hour. All of the ribonuclease solutions from the main peak of the second elution profile up to a maximum of 5 liters were coated onto the Sephadex column. The desalination was carried out using the 50 mM ammonium hydrogen carbonate buffer. The desalination cycle lasted for 90 minutes at which point 5 liters had been collected. The remainder of the cycle was discarded. An elution profile was recorded and the single peak was collected in a 5 liter Schott flask. Traces of the sample of the purified final product were plotted under reduction conditions with beta-mercaptoethanol, and in nonreduced form. The SDS-PAGE studies of the final purified product indicated that only the dimeric form of ribonuclease was present.

In order to place the lyophilized protein into a pharmaceutically useful form, it was necessary to free the ribonuclease dimers of endotoxins. For this purpose, the purified dimers were chromatographed using a Detoxigel column. To obtain a maximum reclamation of the desired ribonuclease dimer material, it was necessary to use buffers in the physiological pH range. This was accomplished through the addition of salt solutions. The enzyme solutions were coated onto the Detoxigel column, and endotoxins in an amount of up to 1,500 E.U./ml were removed. Before and after each cycle, the Detoxigel was regenerated with 1% desoxycholate, followed by a thorough rinsing with water until the endotoxins could no longer be detected in the rinse. The column volume was 750 ml (diameter 9.5 cm, height 14 cm), and the column was equilibrated with 0.1 M ammonium bicarbonate. The endotoxin-free buffer was mixed with sterile water. A ribonucleate solution in a volume of 2 liters was coated onto the gel bed and was left to soak into the gel. The protein was then eluted with the equilibrating buffer and was collected in a sterile beaker. The solution was then passed for lyophilization into a sterile round-bottom flask.

The endotoxin content of the preparation was determined with a diagnostic kit called "Coatest (R)" of the Kabi-Vitrum firm of Munich. This test is based on the activation of Limulus-Amoebocyten-Lysat (LAL) by the endotoxins. The activated LAL separates the yellow dye p-nitroanilin from a chromogenic substate (S-2423), and the reduction in color is measured as a determinant of the endotoxin content at 405 nm by a spectral photometer. The purified ribonuclease dimeric products having below 0.1 ng/mg were passed on to round-bottom flasks and then lyophilized. The resulting product was a lyophilized highly purified ribonuclease dimer solution which could be stored until needed for further use.

Example 2: Enzyne Activity Test

The enzyme activity of the dimeric ribonuclease prepared in the present invention can be tested using an activity test such as described in Kunitz, J. Gen. Physiol. 24:15 (1940). This test is based on the fact that as a result of the influence of ribonuclease on ribonucleic acid, its absorption spectrum is shifted in the UV range toward shorter wavelengths. In a range of 290-305 nm, the concentration of nucleic acid decreases as a result of digestion with ribonuclease. In the initial phase of the reaction, this decrease runs linearly and can be used as a measure for the enzyme activity. In this test, the decrease in the amount of nucleic acid is measured at 300 nm.

The test was carried out at a constant temperature of 25°C, and a temperable cuvette was used so that all the solutions could be treated before use for 5 minutes at 25°C. Measurements were taken at 300 nm using a spectral photometer. A reaction volume was 3 ml and the reaction is carried out in a 3 ml cuvette which includes a layer thickness of 1 cm.

A reaction mixture was prepared using 1.5 ml of a ribonucleic acid solution, 0.05-0.5 ml of the test samples including the ribonuclease fractions, and water added in an amount up to 10 ml. The ribonucleic acid solution was prepared using 80 mg of ribonucleic acid and sodium salt (Boehringer) dissolved in a 50 ml acetate buffer. The acetate buffer was an 0.1 M acetate buffer at pH 5 prepared by mixing 0.57 ml glacial acetic acid with approximately 80 ml of distilled water, adjusted to pH 5 with 1 N NaOH, and filled up to 100 ml with distilled water followed by sterile filtering. The test sample solutions including the ribonuclease dimers were prepared from 5 mg of the ribonuclease dimer sample dissolved in 5 ml of distilled water, diluted so that the measured value was within the favorable range.

The ribonucleic acid and test sample solutions were mixed in a cuvette and the reduction of nucleic acid was tracked for approximately 20 minutes using the spectral photometer spectronic 1001 (Bausch & Lomb). The absorption profile was recorded, and the linear range was observed for about 8 minutes. The reactions were continued at 25°C until the reaction completion, which occurred after approximately 3 hours. The solutions were then measured again for determination of a final value and measurements were repeated twice to get an average value therefrom.

For calculation of the enzyme activity, the extinction of the nucleic acid must be determined at the start of the reaction ($E_o$), and the final extinction ($E_e$) of the reaction at $\Delta$ E/minute of the linear initial range. The dilution of the ribonuclease dimer test sample must be selected so that $\Delta$ E/minute is not greater than 0.007. A blank value is set from a reaction batch made with ribonuclease and water, which must be subtracted from the measured values. An enzyme unit is defined as the quantity of enzyme under which the test conditions cause a drop of 100% per minute of the value of $E-E_e$ for the substrate at 25% C. The greatest possible value for the extinction observed at 300 nm is $E_o-E_e$. The calculation of the specific activity of the enzyme was as follows:

$$\frac{(3 \times \Delta E/min)\ \text{Dilution factor}}{(E_o - E_e)\ \times\ \text{volume (ml) of sample solution}}$$

The dimeric ribonuclease enzymes of the present invention showed significant amounts of enzyme activity.

## Claims

1. A method of preparing a purified ribonuclease dimer product wherein a ribonuclease solution is prepared by adding monomeric ribonuclease to a buffer solution, a suberimidate coupling reagent is added to dimerize the ribonuclease monomers in the ribonuclease solution, and the dimerized ribonuclease solution is separated from contaminations by liquid chromatography, characterized in that

   - the buffer solution is adjusted to a pH of at least 9;
   - the pH is maintained at at least 9 during dimerization;
   - the dimerization reaction is stopped at a given point by lowering the pH to about 7; and
   - at least one additional separation step is carried out by liquid chromatography

2. A method according to claim 1 wherein the monomeric ribonuclease solution is adjusted to a pH of about 10.

3. A method according to claim 2 wherein the monomeric ribonuclease solution is adjusted to pH 10 by adding NaOH.

4. A method according to claim 1 wherein the buffer used in the monomeric ribonuclease solution is comprised of a disodium hydrogen phosphate dihydrate buffer.

5. A method according to claim 1 wherein the coupling reagent is added to the monomeric ribonuclease solution while the solution is maintained at a pH of about 10.

6. A method according to claim 1 wherein the suberimidate coupling reagent comprises dimethyl suberimidate.

7. A method according to claim 1 wherein the dimerization steps are carried out at room temperature.

8. A method according to claim 1 wherein the dimerization steps are carried out while continuously stirring the solution.

9. A method according to claim 1 wherein the dimerization reaction is stopped by addition of HCl.

10. A method according to claim 1 wherein the dimerized ribonuclease solution is concentrated before the liquid chromatography steps, using a tangential flow system.

11. A method according to claim 1 wherein the first liquid chromatography step is carried out using a cross-linked dextran ion exchange resin.

12. A method according to claim 1 wherein the ribonuclease monomer solution comprises 40-60 g of ribonuclease monomer and 4-6 l of buffer, and wherein the coupling reagent is added in an amount of from about 4-6 g.

13. A method according to claim 1 wherein the fractions collected after the first liquid chromatography step are analyzed by gel electrophoresis.

14. A method according to claim 1 wherein the fractions collected following the first liquid chromatography step are lyophilized.

15. A method according to claim 1 wherein fractions collected after the first liquid chromatography step which are primarily monomeric ribonuclease are recycled in the dimerization process.

16. A method according to claim 1 wherein the second liquid chromatography step is carried out using a cross-linked dextran ion exchange resin.

17. A method according to claim 1 wherein the second liquid chromatography step is carried out at a pH of about 5.

18. A method according to claim 17 wherein the pH of the second liquid chromatography step is obtained using a sodium acetate buffer.

19. A method according to claim 1 wherein fractions collected during the second liquid chromatography step are analyzed by gel electrophoresis.

20. A method according to claim 1 wherein a desalination step is carried out after the second liquid chromatography step.

21. A method according to claim 20 wherein the desalination step is carried out using a cross-linked dextran G25 column.

22. A method according to claim 1 further comprising the step of removing endotoxins from the purified ribonuclease dimer product

23. A method according to claim 22 wherein the endotoxins are removed using a Detoxigel column.

## Patentansprüche

1. Verfahren zur Herstellung eines gereinigten Produktes eines Ribonuklease-Dimers, bei dem durch Zugabe einer monomeren Ribonuklease zu einer Pufferlösung eine Ribonukleaselösung hergestellt wird, ein Suberimidat-Kupplungsreagenz hinzugefügt wird, um die Ribonuklease-Monomere in der Ribonukleaselösung zu dimerisieren, und die dimerisierte Ribonukleaselösung durch Flüssig-Chromatographie von Verunreinigungen abgetrennt wird, **dadurch gekennzeichnet, daß**

- die Pufferlösung auf einen pH-Wert von wenigstens 9 eingestellt wird;
- der pH-Wert während der Dimerisation auf wenigstens 9 gehalten wird;
- die Dimerisationsreaktion an einem gegebenen Punkte durch Absenken des pH-Wertes auf etwa 7 abgebrochen wird; und
- wenigstens ein zusätzlicher Abtrennungsschritt mittels Flüssig-Chromatographie durchgeführt wird.

2. Verfahren nach Anspruch 1, bei dem die monomere Ribonukleaselösung auf einen pH-Wert von etwa 10 eingestellt wird.

3. Verfahren nach Anspruch 2, bei dem die monomere Ribonukleaselösung durch Zugabe von NaOH auf einen pH-Wert von 10 eingestellt wird.

4. Verfahren nach Anspruch 1, bei dem der in der monomeren Ribonukleaselösung verwendete Puffer einen Dinatrium-Hydrogenphosphat-Dihydrat-Puffer aufweist.

5. Verfahren nach Anspruch 1, bei dem das Kupplungsreagenz der monomeren Ribonukleaselösung zugegeben wird, während die Lösung auf einem pH-Wert von etwa 10 gehalten wird.

6. Verfahren nach Anspruch 1, bei dem das Suberimidat-Kupp lungsreagenz Dimethyl-Suberimidat aufweist.

7. Verfahren nach Anspruch 1, bei dem die Dimerisationsschritte bei Raumtemperatur durchgeführt werden.

8. Verfahren nach Anspruch 1, bei dem die Dimerisationsschritte unter ständigem Rühren der Lösung durchgeführt werden.

9. Verfahren nach Anspruch 1, bei dem die Dimerisationsreaktion durch Zugabe von HCl abgebrochen wird.

10. Verfahren nach Anspruch 1, bei dem die dimerisierte Ribonukleaselösung vor den Schritten der Flüssig-Chromatographie unter Verwendung eines Systemes mit Tangentialströmung konzentriert wird.

11. Verfahren nach Anspruch 1, bei dem der erste Schritt der Flüssig-Chromatographie unter Verwendung eines quervernetzten Dextran-Ionenaustauschharzes durchgeführt wird.

12. Verfahren nach Anspruch 1, bei dem die monomere Lösung von Ribonuklease 40-60 g Ribonuklease-Monomer und 4-6 1 Puffer aufweist, und bei dem das Kupplungsreagenz in einer Menge von etwa 4-6 g zugefügt wird.

13. Verfahren nach Anspruch 1, bei dem die nach dem ersten Schritt der Flüssig-Chromatographie gesammelten Fraktionen durch Gelelektrophorese analysiert werden.

14. Verfahren nach Anspruch 1, bei dem die nach dem ersten Schritt der Flüssig-Chromatographie gesammelten Fraktionen lyophilisiert werden.

15. Verfahren nach Anspruch 1, bei dem die nach dem ersten Schritt der Flüssig-Chromatographie gesammelten Fraktionen, die hauptsächlich monomere Ribonuklease sind, in den Dimerisationsprozeß rückgeführt werden.

16. Verfahren nach Anspruch 1, bei dem der zweite Schritt der Flüssig-Chromatographie unter Verwendung eines quervernetzten Dextran-Ionenaustauschharzes durchgeführt wird.

17. Verfahren nach Anspruch 1, bei dem der zweite Schritt der Flüssig-Chromatographie bei einem pH-Wert von etwa 5 durchgeführt wird.

18. Verfahren nach Anspruch 17, bei dem der pH-Wert des zweiten Schrittes der Flüssig-Chromatographie unter Verwendung eines Natriumazetatpuffers erhalten wird.

19. Verfahren nach Anspruch 1, bei dem die während des zweiten Schrittes der Flüssig-Chromatographie gesammelten Fraktionen durch Gelelektrophorese analysiert werden.

20. Verfahren nach Anspruch 1, bei dem nach dem zweiten Schritte der Flüssig-Chromatographie ein Entsalzungsschritt durchgeführt wird.

21. Verfahren nach Anspruch 20, bei dem der Entsalzungsschritt unter Verwendung einer quervernetzten Dextran-G25-Kolonne durchgeführt wird.

22. Verfahren nach Anspruch 1, welches ferner den Schritt des Entfernens von Endotoxinen aus dem gereinigten Produkt des Ribonuklease-Dimers umfaßt.

**23.** Verfahren nach Anspruch 22, bei dem die Endotoxine unter Verwendung einer Detoxigel-Kolonne entfernt werden.

## Revendications

**1.** Procédé de préparation d'un dimère de la ribonucléase purifié dans lequel on prépare une solution de ribonucléase en ajoutant de la ribonucléase monomère à une solution tampon, en ajoutant un subérimidate comme agent de couplage pour dimériser les monomères de la ribonucléase dans la solution de ribonucléase, et on sépare la solution de ribonucléase dimérisée des contaminants par chromatographie liquide, caractérisé en ce que :

- la solution tampon est ajustée à un pH d'au moins 9 ;
- le pH est maintenu à au moins 9 au cours de la dimérisation ;
- la réaction de dimérisation est arrêtée à un point donné en abaissant le pH à environ 7 ; et
- au moins un stade de séparation supplémentaire est effectué par chromatographie liquide.

**2.** Procédé selon la revendication 1, dans lequel la solution de ribonucléase monomère est ajustée à un pH d'environ 10.

**3.** Procédé selon la revendication 2, dans lequel la solution de ribonucléase monomère est ajustée à pH 10 en ajoutant NaOH.

**4.** Procédé selon la revendication 1, dans lequel le tampon utilisé dans la solution de ribonucléase monomère est constitué d'un tampon hydrogéno-phosphate disodique dihydrate.

**5.** Procédé selon la revendication 1, dans lequel le réactif de couplage est ajouté à la solution de ribonucléase monomère tandis que la solution est maintenue à un pH d'environ 10.

**6.** Procédé selon la revendication 1, dans lequel le subérimidate servant de réactif de couplage comprend le subérimidate de diméthyle.

**7.** Procédé selon la revendication 1, dans lequel les stades de dimérisation sont effectués à la température ambiante.

**8.** Procédé selon la revendication 1, dans lequel les stades de dimérisation sont effectués tout en agitant la solution en continu.

**9.** Procédé selon la revendication 1, dans lequel la réaction de dimérisation est stoppée par addition de HCl.

**10.** Procédé selon la revendication 1, dans lequel la solution de ribonucléase dimérisée est concentrée avant les stades de chromatographie liquide en utilisant un système à écoulement tangentiel.

**11.** Procédé selon la revendication 1, dans lequel le premier stade de chromatographie liquide est effectué en utilisant une résine échangeuse d'ions de dextrane réticulé.

**12.** Procédé selon la revendication 1, dans lequel la solution de ribonucléase monomère comprend 40 à 60 g de ribonucléase monomère et 4 à 6 litres de tampon, et dans lequel le réactif de couplage est ajouté dans une quantité d'environ 4 à 6 g.

**13.** Procédé selon la revendication 1, dans lequel les fractions recueillies après le premier stade de chromatographie liquide sont analysées par électrophorèse sur gel.

**14.** Procédé selon la revendication 1, dans lequel les fractions recueillies après le premier stade de chromatographie liquide sont lyophilisées.

**15.** Procédé selon la revendication 1, dans lequel les fractions recueillies après le premier stade de chromatographie liquide, qui sont principalement de la ribonucléase monomère, sont recyclées dans le procédé de dimérisation.

**16.** Procédé selon la revendication 1, dans lequel le second stade de chromatographie liquide est effectué en utilisant une résine échangeuse d'ions de dextrane réticulé.

**17.** Procédé selon la revendication 1, dans lequel le second stade de chromatographie liquide est effectué à un pH d'environ 5.

**18.** Procédé selon la revendication 17, dans lequel le pH du second stade de chromatographie liquide est effectué en utilisant un tampon acétate de sodium.

**19.** Procédé selon la revendication 1, dans lequel les fractions recueillies au cours du second stade de chromatographie liquide sont analysées par électrophorèse sur gel.

**20.** Procédé selon la revendication 1, dans lequel un stade de désalinisation est effectué après le second stade de chromatographie liquide.

**21.** Procédé selon la revendication 20, dans lequel le stade de désalinisation est effectué en utilisant une colonne de dextrane G25 réticulé.

**22.** Procédé selon la revendication 1, comprenant en outre le stade d'élimination des endotoxines du dimère de la ribonucléase purifié obtenu.

**23.** Procédé selon la revendication 22, dans lequel les endotoxines sont éliminées en utilisant une colonne de Detoxigel.

*FIG. 1*

EP 0 509 989 B1

*FIG. 2*

EP 0 509 989 B1